**Europäisches Patentamt**

(19) **European Patent Office**

**Office européen des brevets**

(11) Publication number: **0 195 013**
**B1**

(12) **EUROPEAN PATENT SPECIFICATION**

(45) Date of publication of patent specification: **14.03.90**

(51) Int. Cl.⁵: **C 07 C 57/02, B 01 J 21/08**

(21) Application number: **85903496.9**

(22) Date of filing: **25.06.85**

(86) International application number:
**PCT/US85/01137**

(87) International publication number:
**WO 86/00299 16.01.86 Gazette 86/02**

(54) Preparation and use of co-formed catalyst.

(30) Priority: **25.06.84 US 624041**

(43) Date of publication of application:
**24.09.86 Bulletin 86/39**

(45) Publication of the grant of the patent:
**14.03.90 Bulletin 90/11**

(84) Designated Contracting States:
**AT BE CH DE FR GB IT LI LU NL SE**

(56) References cited:
**US-A-2 947 708**
**US-A-3 247 248**
**US-A-3 657 331**
**US-A-3 840 587**
**US-A-3 884 835**
**US-A-4 040 913**
**US-A-4 325 929**

(73) Proprietor: **AMOCO CORPORATION**
**Mail Code 1907 Patents and Licensing**
**Department P.O. Box 5910-A**
**Chicago, IL 60680 (US)**

(72) Inventor: **KADUK, James, A.**
**423 East Chicago Avenue**
**Naperville, IL 60540 (US)**
Inventor: **SMITH, Thomas, G.**
**1344 Chestnut Ridge**
**Naperville, IL 60540 (US)**
Inventor: **HAGEN, Gary, P.**
**721 Duane**
**Glen Ellyn, IL 60137 (US)**
Inventor: **MONTAG, Ruth, A. 799 Royal St.**
**George Drive**
**Apt. 618**
**Naperville, IL 60540 (US)**

(74) Representative: **Ritter, Stephen David et al**
**Mathys & Squire 10 Fleet Street**
**London EC4Y 1AY (GB)**

Courier Press, Leamington Spa, England.

# EP 0 195 013 B1

**Description**

This invention relates to the preparation of a catalyst suitable for the aldo-type condensation of a saturated aliphatic monocarboxylic acid compound and a formaldehyde compound to yield an alpha, betaethylenically unsaturated aliphatic monocarboxylic acid compound of one more carbon atom than the starting saturated aliphatic monocarboxylic acid compound which comprises gelling an aqueous silica colloid and at least one water soluble alkali metal or alkaline earth metal compound.

The literature is replete with disclosures of the reaction of saturated aliphatic monocarboxylic acid compounds with formaldehyde to produce alpha, beta-ethylenically unsaturated aliphatic monocarboxylic acid compounds of one or more carbon atoms than the saturated carboxylic acid compound. The catalysts disclosed are of two general types, those which are water tolerant and those that are relatively water intolerant. In those cases where water intolerant catalysts are employed, it is generally necessary or desirable to use anhydrous reactants. However, for every molecule of alpha, beta-ethylenically unsaturated aliphatic monocarboxylic acid compound produced there is one molecule of water by-product.

By way of example, US—A—3247248 discloses the production of unsaturated monocarboxylic acids by the aldol-type condensation of formaldehyde and acetic or propionic acid. The catalysts used in the Examples are prepared by impregnating a solid carrier, for example sodium alumino-silicate or silica gel, with various inorganic compounds, e.g. calcium acetate, potassium hydroxide, ammonium molybdate or cesium hydroxide.

Irrespective of whether or not the catalyst is water tolerant or not, Kirk-Othmer indicates in Volume 15, 3rd Edition (1981) at pages 364 and 374 that a catalyst for this reaction must provide high selectivity and high conversion and have at least 6 months life. Effective catalysts disclosed include alkali metal or alkaline earth metal aluminosilicates, potassium hydroxide or cesium hydroxide treated pyrogenic silica, alumina and lanthanum oxide. Kirk-Othmer indicates that the data obtained with these catalysts were in short runs and it appears that additional catalyst development is required for this method of producing alpha, beta-ethylenically unsaturated monocarboxylic acid (methacrylic acid) compounds. A careful review of the prior art by us fails to disclose any examples of operations where the reaction has been on stream for more than a day or two. This is not surprising since our experiences have shown that catalyst life is generally low and there is a tendency for coke deposition on the catalyst with the result that the catalyst reactivity drops rapidly. It is not uncommon for coke deposition to reach unacceptable levels in 24 to 48 hours.

Our studies have shown that when using approximately equal molar concentrations of propionic acid and formaldehyde that silica catalysts provide a relatively high degree of conversion and selectivity based on propionic acid. However, our studies have also shown that silica supports tend to degrade over a period of time in the sense that surface area of the catalyst decrease while the average pore size increases, particularly in the presence of water. In other words, silica catalysts tend to be relatively water intolerant. Further, there is a tendency for the cations to be volatilized off over a period of time. As pointed out by Kirk-Othmer there is a need for a catalyst system which permits operation for longer periods of time. While Kirk-Othmer states that the catalyst should have at least 6 months life, we know of no prior art examples that have disclosed condensation reactions of more than 24 to 48 hours. Accordingly, there is a need for a suitable catalyst for the conversion of propionic acid compound to methacrylic acid compound which can be utilized for extended periods of time without substantial degradation during conversion and decoking operations.

Prior to this invention, silica catalysts were prepared in our laboratory by measuring the water absorption of the silica support having the desired porosity and surface area. A suitable alkali metal or alkaline earth metal compound was then dissolved in exactly the amount of water that would fill the pores of the silica support. The aqueous composition was then deposited carefully upon the silica support in a manner such that substantially all of the aqueous catalyst composition was taken up and the silica gel support was dried. For convenience, this technique is referred to as the "incipient wetness" method. As indicated above these catalysts are relatively water intolerant since the surface area of the catalyst decreases during the condensation of propionic acid and formaldehyde while the pore size increases. Further, the more water in the feed, the faster the surface area decreases and the faster the average pore size increases. Although the catalyst tends to be active as the surface area decreases and pore diameter increases, there is a drop off in the activity of the catalyst due to loss of pore structure which is not necessarily dependent upon the loss of the alkali metal or alkaline earth metal cation. As the surface area drops to about 10 to 20 m²/g and the number of pores having a diameter less than 120 nm (1200 Å) decreases, catalyst activity falls off. Accordingly, there is a need for a new silica catalyst which is more water tolerant than those described above. Since it is relatively expensive to provide substantially anhydrous formaldehyde as a reactant and since water is produced in the condensation reaction, it is desirable to employ a water tolerant catalyst in these reactions.

Unless otherwise stated, pore volume, surface area and average pore diameter was determined by BET nitrogen adsorption (desorption test).

The general object of this invention is to provide a new method of producing a water tolerant catalyst suitable for the aldol-type condensation of formaldehyde compounds with saturated aliphatic monocarboxylic acid compounds. Other objects appear hereinafter.

The objects of this invention can be attained by conforming a catalyst by gelling a silica colloid and a suitable alkali metal compound. The silica supported catalysts of this invention are substantially more water tolerant than catalysts prepared by the incipient wetness technique described above. This technique has the additional advantage that it is easier to control decoking the catalysts of this invention. There is a substantially smaller exotherm during decoking and accordingly less chance of destroying the catalyst. It is believed that the reduced exotherm is due to the more even distribution of cations in the silica support.

Briefly, the silica catalysts of this invention can be prepared by gelling an aqueous composition comprising a silica colloid and alkali metal and/or alkaline earth metal cation, drying the composition to remove substantially all of the moisture other than the water of hydration, and calcining.

The alkali metal and/or alkaline earth metal cations of the catalyst can be used in a concentration of 0.001 to .2 equivalents of cation per 100 grams of silica support on a dry solids basis. In general, it is preferred to have from about .004 to .1 equivalents (gram atoms) of cation per 100 grams of silica support on a dry solids basis since the higher the concentration of cation, the lower the temperature needed for condensation of the saturated aliphatic monocarboxylic acid compound and formaldehyde compound and the greater the selectivity and life of the catalyst. The lower the concentration of the cation, the higher the condensation temperature necessary to obtain the desired degree of conversion to alpha, beta-ethylenically unsaturated monocarboxylic acid compound and the lower the selectivity of catalyst and life of the catalyst.

Suitable sources of Group I alkali metal and Group II alkaline earth metal cations include sodium hydroxide, potassium hydroxide, cesium hydroxide, lithium hydroxide, rubidium hydroxide, strontium hydroxide, magnesium hydroxide, lithium phosphate, trisodium phosphate, cesium phosphate, sodium borate, barium hydroxide, sodium carbonate, cesium fluoride, cesium nitrate, etc. Of these, the alkali metal cations are preferred and particularly cesium.

While any commercially available colloidal silica can be used, it is preferred to use commercially available colloidal silicas having an average particle diameter of 4 to 100 nm (40 to 1000 Å), particularly those having a particle diameter of about 5 to 25 nm (50 to 250 Å). The preferred silica supported catalysts have a surface area of 20 to 275 m²/g, a pore volume of .1 to .8 cc/g and an average pore diameter of about 7.5 to 20 nm (75 to 200 Å). Silica catalysts comprising at least one cation of a Group I or Group II metal having a surface area of 20 to 275 m²/g, pore volume of .01 to .8 cc/g and an average pore diameter of 7.5 to 20 nm (75 to 200 Å) have relatively high activity (% conversion and selectivity) and relatively long life. Pore volume, surface area and average pore diameter are interdependent variables. Other things being equal, holding one variable constant, as the surface area increases, pore volume increases; as the surface area increases, average pore diameter decreases; and as the pore volume increases, average pore diameter increases. It is critical in S.N. 624,040 that the catalyst satisfy each of the pore volume, surface area and average pore diameter requirements. For example, if the catalyst has a porosity greater than .8 cc/g, the catalyst lacks the strength to resist attrition necessary for use over extended periods of time. If the porosity is less than .1 cc/g, the surface area is too low and/or the average pore diameter is too high. However, the catalyst loses activity as it loses pores having a diameter under 120 nm (1200 Å). Accordingly, it is preferred to use a catalyst having a substantially smaller average pore diameter to insure that the catalyst has adequate life. If the average pore diameter of the starting catalyst is substantially higher than 20 nm (200 Å), there is a substantial decrease in the life of the catalyst. Pore diameters of at least 7.5 nm (75 Å) are necessary in order to permit gas diffusion of the reactants and reaction products.

In somewhat greater detail, the silica supports of this invention can be prepared by forming an aqueous composition comprising about 10 to 60% by weight colloidal silica on a dry basis and alkali metal and/or alkaline earth metal cation. The colloidal silica is gelled by adjusting the pH to a range of about 3 to 10, preferably about pH 6.0 to about 9.0, preferably with alkali metal or alkaline earth metal cations. Salts such as $NH_4NO_3$ can be used to accelerate gelation. While silica hydrogels can be aged for two weeks or more, aging seems to have no effect on the properties of the catalyst and accordingly, aging is not necessary. The composition is then dried by any suitable means, such as in a microwave oven, to constant weight and apparent dryness, e.g., about 4 to 5% moisture on a dry solids basis. Apparently only the water of hydration is retained by the silica gel after drying to constant weight. The silica gel is then calcined at about 300 to 800°C, preferably about 300 to 600°C. At calcination temperatures above about 800°C, there is a tendency for the surface area to go down, the pore volume to go down and the pore diameter to go up. However, these catalysts are substantially more water tolerant than silica catalysts produced by the incipient wetness technique.

The catalysts of this invention can be used for the aldol-type condensation of saturated aliphatic monocarboxylic acid compounds to alpha, beta-ethylenically unsaturated monocarboxylic acid compounds of one more carbon atom than the starting saturated aliphatic carboxylic acid compound. Suitable aliphatic monocarboxylic acid compounds that can be converted in the aldol-type condensation reaction include acetic acid, propionic acid, methyl acetate, methyl propionate, ethyl propionate, acetonitrile, propionitrile, etc. The preferred saturated monocarboxylic acid compounds are propionic acid compounds and particularly propionic acid since the catalyst of this invention has been designed primarily for large scale production of methacrylic acid.

While any suitable source of formaldehyde compound can be used, such as formalin, paraformaldehyde, methanolic formaldehyde, trioxane, etc., it is preferred to use substantially anhydrous formaldehyde, particularly cracked monomeric, gaseous, substantially anhydrous formaldehyde.

3

Briefly, an alpha, beta-ethylenically unsaturated monocarboxylic acid compound can be prepared by condensing under vapor phase conditions a saturated aliphatic monocarboxylic acid compound and formaldehyde compound in the presence of a coformed silica gel catalyst preferably one comprising at least one cation of Group I or Group II metal and a silica gel support, said support having a surface area of 20 to 275 $m^2$/g, a pore volume of .1 to .8 cc/g and an average pore diameter of 7.5 to 20 nm (75 to 200 Å).

This reaction can be carried out advantageously in the presence of a water-immiscible hydrocarbon or halohydrocarbon diluent of from about 6 to 12 carbon atoms. Use of a diluent is advantageous in increasing the percent yield by approximately 10% (e.g. from 30 to 33%). Further, as explained below, the diluent has additional functions in the overall unitary process for producing methacrylic acid from propionic acid. Suitable diluents include n-hexane, n-heptane, n-octane, 2-ethylhexane, n-decane, n-dodecane, o,p,m-xylene, benzene, toluene, etc. The concentration of diluent can range from about 10 to 50% by weight of the reactants in the main reactor.

The molar ratio of monocarboxylic acid compound to formaldehyde can range from 25:1 to 1:25. However, best results with this catalyst in the production of methacrylic acid can be obtained using a molar ratio of propionic acid compound to formaldehyde of from about .5—2.0 to 1. In general, the lower the molar ratio, the higher the percent conversion based upon the amount of propionic acid converted.

While the aldol-type condensation can be carried out at about 280 to 500°C, it is preferred to operate at about 280 to 350°C since selectivity goes up as the reaction temperature goes down. It is to be noted that (1) the amount of undesirable unsaturated cyclic ketone by-product which is a catalyst for the polymerization of alpha, beta-ethylenically unsaturated monocarboxylic acids can be reduced from 4 mol % based on starting propionic acid compound at 390°C to approximately 2.5 mol % at 350°C or less (about 1% at 325°C) or (2) over a 80-day period of alternate 24-hour periods of condensation followed by 24-hour periods of decoking that the loss of cation can be reduced from over 75% at 390°C with attendant loss of catalytic activity to about 10% at 350°C with constant activity.

In somewhat greater detail, the unitary process for the production of methacrylic acid comprises (1) feeding propionic acid and formaldehyde compound to a reactor containing the silica catalyst of this invention, (2) condensing under vapor phase conditions formaldehyde and propionic acid to produce a composition comprising water, formaldehyde, propionic acid and methacrylic acid, (3) distilling said reaction product to remove water, unreacted formaldehyde and at least some of the propionic acid from the reaction product, (4) passing an entraining agent comprising a water immiscible diluent of from 6 to 12 carbon atoms to the distillation column to remove water and at least some of the formaldehyde overhead.

In a still more preferred version of this process, a side draw is located at least part way up the distillation column to remove a composition comprising substantially all of the propionic acid and at least some of the formaldehyde. The use of a side draw is disclosed and claimed in WO/8600236. As pointed out in WO8600236 the side draw facilitates the removal of part of the formaldehyde from the aqueous mixture going overhead and thereby precludes polymerization of formaldehyde at the top of the distillation column thereby eliminating or reducing the possibility of plugging at the top of the distillation column. Irrespective of whether a side draw is employed or not, it is contemplated that the formaldehyde is recovered from the aqueous formaldehyde taken overhead by reacting the aqueous formaldehyde with an alcohol of from about 6 to 12 carbon atoms to form a hemiacetal, distilling water from the hemiacetal and then cracking the substantially anhydrous hemiacetal to recover the formaldehyde. The formaldehyde is advantageously separated from the alcohol by adding a water immiscible diluent at the top of the column in order to facilitate the removal of the formaldehyde from the alcohol used to form the hemiacetal. The formaldehyde and diluent are then recycled to the main reactor.

The process of this invention can be carried out at a weight hourly space velocity of about .1 to 10, preferably .5 to 6.5. In general, the lower the weight hourly space velocity, the lower the reaction temperature necessary. The higher the weight hourly space velocity the higher the reaction temperature necessary.

The catalysts of this invention are preferably decoked after about 12 to 72 hours on stream. In order to prevent sintering of the silica and/or loss of cation, dilute oxygen (1 to 5% by volume and 95 to 99% by volume inert gas) is contacted with the catalyst bed at about 232 to 343°C (450 to 650°F), preferably 232 to 288°C (450 to 550°F), while holding the exotherm to about 5.5 to 16.5°C (10 to 30°F), increasing the oxygen content incrementally while holding the exotherm to about 5.5 to 16.6°C (10 to 30°F) until there is no exotherm with a mixture of 20% oxygen and 80% inert gas, e.g. air, followed by incrementally raising the temperatures by 14 to 42°C (25 to 75°F) and controlling the exotherm to about 10 to 30°F until decoking is completed at about 343 to 427°C (650 to 800°F).

In the examples that follow, percent conversion, percent yield and percent selectivity are all based on propionic acid (PA) unless otherwise stated.

It should be noted that in Examples II to IX where mini-reactors were used, the reaction temperature had to be sufficiently high to crack trioxane to monomeric formaldehyde (390°C). Accordingly, it is anticipated that yields and selectivities will be better when operating with monomeric formaldehyde at lower temperatures.

Example I

This Example illustrates the production of methacrylic acid in a pilot plant reactor using a coformed

4

cesium phosphate silica gel catalyst having a surface area of 119 m²/g, porosity of .604 cc/g and an average pore diameter of 16.8 nm (168 Å) containing 1.97 weight percent cesium based on the dry weight of the silica. A slurry of 29 parts by weight paraformaldehyde, 106 parts by weight propionic acid (PA:FA molar ratio 3:2) and 47 parts by weight heptane was continuously vaporized to thermally decompose the paraformaldehyde to monomeric formaldehyde at 204°C (400°F). The composition was then conveyed to a reactor system comprising 25 mm (1″) outside diameter by 21 mm (.834″) inside diameter by 1.73 m (6′) Inconel tube equipped with 6 mm (0.25″) outside diameter thermowell having a 1.2 m (4′) long catalyst zone containing 200 grams of catalyst and on each side of the catalyst a 0.3 m (1′) zone of Denstone packing. The thermowell was equipped with thermocouples inserted at 0.15 m (6″) intervals and electrical heating means were positioned along the reactor. Conversion of propionic acid and formaldehyde to methacrylic acid was carried out for 24 hours while maintaining the pilot plant reactor at 660°F (350°C), 10 psig and a weight hourly space velocity of 1.55. The reaction product was collected in a heat exchanger and condensed. After 24 hours, feed to the reactor was turned off and the reactor temperature was reduced to 288°C (550°F). Two percent oxygen in nitrogen was added slowly to the reactor in order to limit the exotherm during decoking to about 11°C (20°F). After that exotherm passed, the oxygen content was increased to 10% and after that exotherm was limited to 11°C (20°F), the nitrogen-oxygen mixture was replaced with air. After each exotherm passed, the temperature of the reactor was raised by 28°C (50°F) increments by closely controlling the exotherm until the reactor was at 371°C (700°F). This decoking process typically takes 2 to 4 hours. Air was continuously flowed through the reactor at 371°C (700°F) for a total of 24 hours. The air was turned off, the reactor temperature was reduced to 349°C (660°F) and the condensation of propionic acid and formaldehyde was begun. The sequential condensation and decoking operation was carried out for 80 days of which 40 days was methacrylic acid production and 40 days decoking. The molar ratio of PA:FA was varied from 1.5:1 to 1.34:1. The physical properties of the catalyst before and after the 80 days onstream is set forth below in Table I. The yields after the first day and the average for the first 66 days also are set forth below in Table I.

TABLE I

| | Initial catalyst and first day analysis of products | Final catalyst and avg. analysis of products |
| --- | --- | --- |
| Cesium content | 1.97 wt.% | 1.76 wt.% |
| Surface area | 119 m²/g | 59 m²/g |
| Pore volume | .604 cc/g | .570 cc/g |
| Average pore diameter | 16.8 nm (168 Å) | 30.2 nm (302 Å) |
| Molar ratio of PA:FA | 1.5 | 1.34 |
| Conversion based on PA | 32.1% | 32.1% |
| Selectivity based on PA | 91.3% | 85.9% |
| MA/PA yield | 29.3% | 27.6% |
| Conversion based on FA | 51.9% | 50.0% |
| Selectivity based on FA | 84.9% | 74.2% |
| MA/FA yield | 44.1% | 37.1% |

The catalyst employed in this Example was prepared by intensely stirring a solution of 10302.9 grams Nalcoag, 1034-A colloidal silica (34% solids, 20 nm (200 Å) particle diameter) and a solution of 111.66 grams cesium phosphate (having an average of 5 molecules of water per mol) in 500 cc deionized water. After intense stirring for 10 minutes, a solution of 100 grams ammonium nitrate in 150 grams deionized water was added to the sol and the mixture was stirred for 2 minutes at which time it began to thicken. The silica gel was permitted to harden after standing at room temperature overnight. The gel was dried in a microwave oven to constant weight, sized to 20 to 40 mesh and calcined according to the following increments: 2 hours at 165°C, followed by increasing the temperature gradually to 540°C over 4 hours and then maintaining at 540°C for an additional 8 hours. All of the steps were carried out in flowing air.

Substantially the same results can be obtained by replacing the cesium phosphate with 2% by weight cesium as the hydroxide or carbonate.

Example II

This Example illustrates that coformed catalysts are more water tolerant than catalysts prepared by the incipient wetness technique. Each of these catalyst runs was carried out in a vertical laboratory 0.35 to 0.46 m (14 to 18″) long quartz mini-reactor having an inside diameter of about 12.5 mm (1/2″) equipped with a thermowell having a diameter of 4.8 to 6.5 mm (about 3/16 to 1/4″). Each of the reactors contained a quartz spun plug to support approximately a 63 to 76 m (2-1/2 to 3″) bed of catalyst weighing approximately 2 to 3 grams. The mini-reactor was enclosed in an electrical furnace maintained at about 390°C. A liquid feed comprising either anhydrous 3:2 molar ratio of propionic acid to trioxane or aqueous mixture comprising 3:2 molar ratio of propionic acid and formalin (23% by weight water in the feed) was dripped into the vertical reactor and vaporized therein. In order to achieve comparable initial conversion, the weight hourly space velocities were adjusted as needed prior to the start of the run. Sparge samples were taken after every 24 hours on feed and the catalyst was decoked for 23 hours at 390°C after every 48 hours on feed. The coformed catalyst used with the anhydrous feed was the catalyst prepared in Example I of this application and the weight hourly space velocity was 2.3. The catalyst for the aqueous coformed catalyst feed contained 1.6% by weight cesium as cesium phosphate was prepared in the same manner as the catalyst in Example I. The weight hourly space velocity of this catalyst was 1.6. The catalyst prepared by the incipient wetness technique contained 2.1% by weight cesium as cesium phosphate on silica gel support prepared as follows. A sample of Ludox AS-40 Brand colloidal silica, 40 wt.% silica, was treated by the dropwise addition of concentrated nitric acid until the pH changed from about 10.5 to 3.0. The pH was then raised to about 6.0 by the dropwise addition of concentrated ammonium hydroxide. The mixture was stirred for 8 hours, at which time a thick gel had formed. The gel was dried overnight at 120°C, then crushed and sized to 18—40 mesh. The material was washed three times to remove sodium by being submerged in 0.10 N nitric acid for fifteen minutes at 75°C. It was then washed five times with deionized water at 50°C and dried overnight in an oven at 120°C. The incipient wetness technique was used to impregnate the dried silica support with an aqueous solution of cesium phosphate. The catalyst was then dried overnight at 120°C and calcined as described in Example I. The resulting catalyst had a BET surface area of 96 m²/g, pore volume of 0.4971 cc/g, and average pore diameter of 18 nm (180 Å). The weight hourly space velocity was 1.6 for the anhydrous feed and .99 for the aqueous feed. The results are set forth below in terms of grams of propionic acid contacted per gram of catalyst on a dry solids basis.

TABLE 2A

| Feed g PA/g catalyst | Coformed catalyst | | | | Incipient wetness catalyst | | | |
| --- | --- | --- | --- | --- | --- | --- | --- | --- |
| | Anhydrous | | Aqueous | | Anhydrous | | Aqueous | |
| | Conversion | Yield | Conversion | Yield | Conversion | Yield | Conversion | Yield |
| 10—20 | | | | | 37 | 22 | 35 | 16 |
| 40—60 | 37 | 22 | 37 | 21 | | | | |
| 75—85 | 35 | 22 | | | 37 | 22 | 32 | 15½ |
| 105 | | | 35 | 23 | | | | |
| 140—160 | 35 | 21 | 40 | 22 | 37 | 22 | 22½ | 11 |
| 185 | | | | | 37½ | 22 | 18 | 10 |
| 195 | | | 39 | 21 | | | | |
| 210 | 34 | 20 | | | | | | |
| 225 | | | | | | | 11 | 10 |
| 240—250 | | | 37 | 19 | 37 | 21 | | |
| 290 | | | 28 | 17 | | | | |
| 310—330 | 25 | 18 | 28 | 14 | 34 | 22 | | |

6

The change in the BET pore volume distributions for the used coformed and incipient wetness catalysts are summarized in Table 2B. This example illustrates that coformed catalysts are substantially more water tolerant than catalysts prepared by the incipient wetness technique.

TABLE 2B

|  | Coformed | | Incipient wetness catalyst | |
| --- | --- | --- | --- | --- |
|  | Anhydrous | Aqueous | Anhydrous | Aqueous |
| Surface area ($m^2/g$) | 67 | 57 | 66 | 16 |
| Pore vol. (cc/g) | 0.6173 | 0.5501 | 0.5048 | 0.0729 |
| Avg. pore diameter nm (Å) | 26.2 (262) | 31 (310) | 22.8 (228) | 30.4 (304) |

Example III

This Example illustrates the preparation and use of other coformed silica gel catalysts. Unless indicated otherwise, the catalysts were prepared in the manner described in Example I and tested in a mini-reactor in the manner described in Example II for the anhydrous feed. The pore size, pore volume and surface area were similar to those of the catalysts of Example I when all of the cation material set forth in Table 3 was water soluble.

TABLE 3

| Description | %C | %Y | %S | WHSV |
| --- | --- | --- | --- | --- |
| 2.1% Cs as $Cs_3PO_4$ | 41 | 29 | 70 | 2.5 |
| 1.7% Cs as $Cs_2ZrO_3$ | 43 | 27 | 62 | 2.5 |
| 1.7% Cs as $Cs_2TiO_3$ | 48 | 27 | 55 | 2.5 |
| 2.2% La as $La(NO_3)_3$ | 34 | 15 | 43 | 2.6 |
| 0.73% La as $La(NO_3)_3$+ 2.1% Cs as CsF | 42 | 24 | 56 | 2.6 |
| 0.73% La as $La(NO_3)$+ 2.1% Cs as CsOH | 42 | 24 | 58 | 2.6 |
| 2.1% Cs as $Cs_3PO_4$+ 1.0% Zn as $Zn(NO_3)_2$ | 28 | 18 | 65 | 2.5 |
| 0.51% K as $K_4P_2O_7$ | 31 | 24 | 77 | 2.0 |
| 1.7% Cs as $Cs_2CO_3$ | 42 | 28 | 66 | 2.5 |

C=Conversion.
Y=Yield.
S=Selectivity.
WHSV=Weight Hourly Space Velocity.

Example IV

Example III was repeated except that samples were taken for analysis 24 hours after completion of the first decoking step.

TABLE 4

| Description | %C | %Y | %S | WHSV |
|---|---|---|---|---|
| 1.7% Cs as CsF | 36 | 23 | 63 | 2.76 |
| 1.7% Cs as CsNO$_3$ | 40 | 25 | 62 | 2.18 |
| 1.7% Cs as Cs$_2$SO$_4$ | 36 | 21 | 58 | 2.29 |
| 1.7% Cs as CsOH | 38 | 24 | 64 | 2.12 |
| 1.7% Cs as CsOH | 40 | 26 | 64 | 2.54 |
| 1.6% Cs as Cs$_2$CO$_3$ | 36 | 24 | 67 | 2.14 |
| 2.0% Cs as Cs$_3$PO$_4$ | 35 | 22 | 61 | 2.26 |
| 2.0% Cs as Cs$_3$PO$_4$ | 37 | 23 | 62 | 2.13 |
| 2.0% Cs as Cs$_3$PO$_4$ | 36 | 22 | 59 | 2.02 |
| 1.6% Cs as Cs$_3$PO$_4$ | 40 | 26 | 65 | 2.57 |
| 1.6% Cs as Cs$_3$PO$_4$+ 10% Ba as Ba$_3$(PO$_4$)$_2$ | 39 | 24 | 62 | 1.50 |
| 2.4% Cs as Cs$_3$PO$_4$+ 10% Li as Li$_3$PO$_4$ | 36 | 22 | 61 | 1.17 |
| 1.7% Cs as CsOAC | 40 | 25 | 64 | 2.54 |

C=Conversion.
Y=Yield.
S=Selectivity.
WHSV=Weight Hourly Space Velocity.

Example V

This Example sets forth the physical properties of coformed cesium catalysts and coformed silica gel catalysts having cation material of the preceding two examples.

TABLE 5

| Description | Surface area in m$^2$/g | Pore volume cc/g | Pore radius in nm (Å) |
|---|---|---|---|
| 1.3% Cs as Cs$_2$SO$_4$ | 164 | 0.5195 | 5.6 (56) |
| 1.9% Cs as CsNO$_3$ | 161 | 0.5041 | 5.6 (56) |
| 0.78% Cs as CsNO$_3$ | 140 | 0.5183 | 7.0 (70) |
| 1.6% Cs as CsOAC | 161 | 0.6323 | 7.0 (70) |
| 1.6% Cs as Cs$_2$CO$_3$ [a] | 137 | 0.7338 | 9.3 (93) |
| 1.7% Cs as CsOH [b] | 135 | 0.6695 | 9.1 (91) |
| 1.6% Cs as Cs$_3$PO$_4$ | 136 | 0.584 | 7.7 (77) |
| 2.0% Cs as Cs$_3$PO$_4$ | 119 | 0.604 | 8.4 (84) |
| 1.0% Cs as CsF | 117 | 0.6893 | 10.2 (102) |

[a]—After 38 days on stream surface area was 119 m$^2$/g, 0.755 cc/g and 9.7 nm (97 Å) average pore radius.
[b]—After 13 days on stream surface area was 117 m$^2$/g, .683 cc/g and 9.7 nm (97 Å) average pore radius.

Example VI

This Example illustrates the preparation of a silica gel catalyst from a colloidal sol having 5 nm (50 Å) diameter particles. The coformed silica gel catalyst was prepared in the same manner as in Example I except that Nalco 2326 containing 5 nm (50 Å) diameter particles and 14.5% by weight solids was used in place of the Nalco 1034A. Gelation was carried out by adjusting the pH to 9 with nitric acid. After calcination the catalyst had a BET surface area of 275 m²/g, a pore volume of .753 cc/g and an average pore diameter of 7.8 nm (78 Å). The catalyst was run in a mini-reactor under the conditions set forth in Example II for the anhydrous feed. After .9 days on stream at 2.06 weight hourly space velocity, the yield was 21%, percent propionic acid conversion 44% and percent propionic acid selectivity 48%. After 3.1 days on stream and one regeneration of the catalyst after 48 hours on stream, the percent yield was 19%, percent propionic acid conversion 40% and percent propionic acid selectivity 48%.

Example VII

The catalyst preparation of Example I was repeated using (1) PQ 2034DI and (2) PQ 2040NH₄, in place of the Nalco 1034A. The silica gel catalysts had, respectively, (1) a BET surface area of 137 m²/g, a pore volume of .602 cc/g and an average pore diameter of 14.8 nm (148 Å) and (2) 146 m²/g BET surface area, a pore volume of .621 cc/g and 14.2 nm (142 Å) diameter. Each of these catalysts were used in the mini-reactor under the conditions set forth in Example II for anhydrous feed. Percent yield was approximately 23%, percent conversion about 41% and percent selectivity about 56% after one and three days on stream.

Example VIII

This Example illustrates the preparation of coformed catalyst from a silica colloid having a diameter of 100 nm (1000 Å). The preparative process of Example I was repeated using PQ sol 9950 which yielded after calcination a catalyst having a surface area of 51 m²/g, pore volume .417 cc/g, and an average pore diameter of 34 nm (340 Å). This catalyst was then run in a mini-reactor in the manner described in Example II. After one day on stream the propionic acid base yield was 22%, propionic acid base conversion 36%, and propionic acid base selectivity was 62%. After three days on stream the yield dropped to 16%, conversion to 30% and selectivity to 55%.

Example IX

This Example illustrates variations in physical properties of silica gel catalysts due to variations in calcination temperature. The catalyst preparation method of Example I was repeated except that the calcination temperature was incrementally raised to the temperatures set forth below in Table 6 and held at said temperature for eight hours. The catalysts were then tested in a mini-reactor in the manner described in Example II.

TABLE 6

| Calcination temp. | 540°C | 700°C | 800°C | 850°C | 900°C |
|---|---|---|---|---|---|
| Surface area in m²/g | 115 m²/g | 57 m²/g | 20 m²/g | 10 m²/g | 1.4 m²/g |
| APD in nm (Å) | 17.4 (174 Å) | 27.6 (276 Å) | 89 (890 Å) | >90 (>900 Å) | >90 (>900 Å) |
| PV in cc/g | .58 cc/g | .55 cc/g | .32 cc/g | .05 cc/g | .02 cc/g |
| % PA selectivity | 62% | 56% | 65% | 61% | 36% |
| % PA conversion | 33% | 32% | 31% | 27% | 25% |
| % Yield on PA | 22% | 18% | 21% | 16% | <10% |

The table clearly shows that as calcination temperature increases, the surface area of the catalyst goes down, average pore diameter goes up and pore volume goes down. Further, as the surface area goes below 20 m²/g and the average pore diameter increases, the percent yield based on propionic acid decreases.

**Claims**

1. A method of producing a coformed catalyst suitable for use in the aldol-type condensation of saturated aliphatic monocarboxylic acid compound and formaldehyde compound comprising at least one cation of a Group I metal and a silica support, characterised in that said coformed catalyst is produced by gelling an aqueous composition comprising colloidal silica and at least one cation of a Group I metal, followed by drying and then calcining the composition.

2. The process according to Claim 1 wherein the colloidal silica has an average diameter of 5 to 25 nm (50 to 250 Å).

3. The process according to Claim 1 or Claim 2 wherein the aqueous composition comprises 10 to 60% silica solids and the composition is gelled by adjusting the pH to a range of 3 to 10.

4. The process according to any preceding claims wherein said silica is gelled at pH 6.0 to 9.0.

5. The process according to any preceding claim wherein the cation is present in the aqueous composition in a concentration of 0.004 to 0.1 equivalents per 100 parts by weight silica on a dry solids basis.

6. The process according to any preceding claim wherein said Group I metal cation comprises cesium.

7. The process according to any preceding claim wherein calcining is carried out at 300 to 800°C.

8. A process for producing an $\alpha$-, $\beta$-ethylenically unsaturated monocarboxylic acid compound by the aldol-type condensation of a saturated aliphatic monocarboxylic acid compound and a formaldehyde compound under vapour phase conditions in the presence of a coformed catalyst produced by the process of any preceding claim.

9. The process according to Claim 9 wherein said saturated aliphatic monocarboxylic acid compound comprises propionic acid.

**Patentansprüche**

1. Verfahren zur Herstellung eines cogebildeten Katalysators, der sich für eine Anwendung bei der Aldol-Kondensation einer gesättigten aliphatischen Monocarbonsäureverbindung und einer Formaldehydverbindung eignet und der wenigstens ein Kation eines Metalls aus der Gruppe I des Periodensystems enthält, dadurch gekennzeichnet, daß dieser cogebildete Katalysator durch Gelierung einer wäßrigen Zusammensetzung aus kolloidalem Siliciumdioxid und wenigstens einem Kation eines Metalls aus der Gruppe I des Periodensystems, anschließende Trocknung und nachfolgende Calcinierung der Zusammensetzung hergestellt wird.

2. Verfahren nach Anspruch 1, dadurch gekennzeichnet, daß das kolloidale Siliciumdioxid einen mittleren Durchmesser von 5 bis 25 nm (50 bis 250 Å) hat.

3. Verfahren nach Anspruch 1 oder 2, dadurch gekennzeichnet, daß die wäßrige Zusammensetzung 10 bis 60% Siliciumdioxidfeststoffe enthält und daß diese Zusammensetzung durch Einstellung des pH-Werts auf einem Bereich von 3 bis 10 in ein Gel überführt wird.

4. Verfahren nach einem der vorhergehenden Ansprüche, dadurch gekennzeichnet, daß das Siliciumdioxid bei einem pH-Wert von 6,0 bis 9,0 in ein Gel überführt wird.

5. Verfahren nach einem der vorhergehenden Ansprüche, dadurch gekennzeichnet, daß das Kation in der wäßrigen Zusammensetzung in einer Konzentration von 0,004 bis 0,1 Äquivalenten auf 100 Gewichtsteile Siliciumdioxid, bezogen auf die trockenen Feststoffe, vorhanden ist.

6. Verfahren nach einem der vorhergehenden Ansprüche, dadurch gekennzeichnet, daß das Metall aus der Gruppe I des Periodensystems Cäsium ist.

7. Verfahren nach einem der vorhergehenden Ansprüche, dadurch gekennzeichnet, daß die Calcinierung bei 300 bis 800°C durchgeführt wird.

8. Verfahren zur Herstellung einer $\alpha,\beta$-ethylenisch ungesättigten Monocarbonsäureverbindung durch Aldol-Kondensation einer gesättigten aliphatischen Monocarbonsäureverbindung und einer Formaldehydverbindung unter den Bedingungen einer Dampfphase in Gegenwart eines cogebildeten Katalysators, der nach dem Verfahren eines der vorhergehenden Ansprüche hergestellt worden ist.

9. Verfahren nach Anspruch 8, dadurch gekennzeichnet, daß die gesättigte aliphatische Monocarbonsäureverbindung Propionsäure ist.

**Revendications**

1. Procédé de production d'un catalyseur co-formé approprié pour une utilisation dans la condensation de type aldol d'un composé d'acide monocarboxylique aliphatique saturé et d'un composé du formaldéhyde comprenant au moins un cation d'un métal du groupe I et un support de silice, caractérisé en ce que ledit catalyseur co-formé est produit par gélification d'une composition aqueuse comprenant de la silice colloïdale et au moins un cation d'un métal du groupe I, suivie par le séchage puis la calcination de la composition.

2. Procédé selon la revendication 1, dans lequel la silice colloïdale a un diamètre moyen de 5 à 25 nm (50 à 250 Å).

3. Procédé selon la revendication 1 ou la revendication 2, dans lequel la composition aqueuse comprend 10 à 60% de solides de silice et la composition est gélifée par réglage du pH dans une gamme de 3 à 10.

4. Procédé selon l'une des revendications précédentes, dans lequel ladite silice est gélifiée à un pH de 6,0 à 9,0.

5. Procédé selon l'une des revendications précédentes, dans lequel le cation est présent dans la composition aqueuse en une concentration de 0,004 à 0,1 équivalent pour 100 parties en poids de silice sur une base de solides secs.

6. Procédé selon l'une des revendications précédentes, dans lequel ledit cation de métal du groupe I comprend du césium.

7. Procédé selon l'une des revendications précédentes, dans lequel la calcination est réalisée entre 300 et 800°C.

8. Procédé de production d'un composé d'acide monocarboxylique α-, β-éthléniquement insaturé par la condensation de type aldol d'un composé d'acide monocarboxylique aliphatique saturé et d'un composé du formaldéhyde dans des conditions de phase gazeuse en présence d'un catalyseur co-formé produit par le procédé selon l'une des revendications précédentes.

9. Procédé selon la revendication 9, dans lequel ledit composé d'acide monocarboxylique aliphatique saturé comprend l'acide propionique.